# EUROPÄISCHE PATENTSCHRIFT

(11) **EP 2 918 630 B1**
(45) Veröffentlichungstag und Bekanntmachung des Hinweises auf die Patenterteilung: **20.06.2018**
(21) Anmeldenummer: 15158975.1
(22) Anmeldetag: 13.03.2015
(51) Int. Cl.: C08J 7/04, C09C 3/10, C08J 7/06, C09C 1/00

(54) **GLITTER UND VERFAHREN ZU DESSEN HERSTELLUNG**
GLITTER AND METHOD FOR ITS PRODUCTION
PAILLETTES ET SON PROCÉDÉ DE FABRICATION

(30) Priorität: 14.03.2014 DE 102014204819
(43) Veröffentlichungstag der Anmeldung: 16.09.2015
(73) Patentinhaber: Sigmund Lindner GmbH, 95485 Warmensteinach (DE)
(72) Erfinder: Pschierer, Erwin, 95506 Kastl (DE)
(74) Vertreter: Isarpatent

(56) Entgegenhaltungen:
- DE-A1-102005 018 617
- DE-A1-102010 001 971
- DE-A1-102011 087 740
- DE-A1-102013 105 877
- DE-U1-202012 102 110

## Beschreibung

Die Erfindung betrifft einen Glitter umfassend eine in Wesentlichen antimonfreie Folie auf Basis eines Polyesters, welche mit einem Metall, vorzugsweise Aluminium, beschichtet ist, sowie ein Verfahren zur Herstellung desselben.

### Stand der Technik

Glitter finden vielfach Anwendung zur Erzeugung eines glitzernden Oberflächeneffektes und werden unter anderem und insbesondere in kosmetischen Artikeln eingesetzt. Zur Herstellung derartiger Glitter werden Folien oder Filme aus Kunststoff verwendet, die mittels eines Schneidvorganges in einzelne, vergleichbar kleine Partikel geschnitten werden.

Ein beispielhaftes Verfahren zur Herstellung solcher Glitter ist in der DE 102010001971 A1 offenbart. Hierin werden Glitter offenbart, die von allen Seiten beschichtet wird.

Es besteht jedoch weiterhin ein Bedarf an Glitter, welche eine verbesserte Brillanz aufweisen. Weiterhin ist es wünschenswert, Glitter mit einem verbesserten Skin-Feeling bereitzustellen, also solche, die bei einer Anwendung in kosmetischen Produkten ein weicheres und angenehmeres Gefühl auf der Haut hervorrufen. Darüber hinaus besteht ein Bedarf an Glitter, welche bei einem Beschichtungsverfahren eine verbesserte, gleichmäßigere und glattere Beschichtung ermöglichen.

Der Erfindung liegt daher die Aufgabe zugrunde, Glitter bereitzustellen, welche eine erhöhte Brillanz und ein verbessertes Skin-Feeling aufweisen. Weiterhin ist es Aufgabe der Erfindung, Glitter bereitzustellen, welche bei einer Beschichtung eine gleichmäßigere und glattere Beschichtung ermöglichen und welche aufgrund Ihrer guten Verträglichkeit in Kosmetikprodukten eingesetzt werden können.

### Zusammenfassung der Erfindung.

Bisher wurde für die Herstellung von Glitter auf Basis von Polyestern stets ein Katalysator auf Antimonbasis verwendet. Es hat sich überraschend gezeigt, dass die Aufgabe der Erfindung, also Glitter mit verbesserter Brillanz und verbessertem Skin-Feeling, sowie Glitter mit verbessertem Beschichtungsverhalten, gelöst werden kann, indem bei der Herstellung von Glitter auf Polyesterbasis auf solche antimonhaltigen Katalysatoren verzichtet wird.

Die vorliegende Erfindung betrifft somit Glitter umfassend eine Folie auf Basis eines Polyesters, welche mit einem Metall, vorzugsweise Aluminium, beschichtet ist, wobei die Folie im Wesentlichen antimonfrei ist. Weiterhin betrifft die Erfindung ein Verfahren zur Herstellung eines Glitters, bei dem eine Folie auf Basis eines Polyesters mit einem antimonfreien Katalysator hergestellt wird, mit einem Metall, vorzugsweise Aluminium, beschichtet wird und die Folie danach geschnitten wird, oder die Folie geschnitten wird und danach mit einem Metall, vorzugsweise Aluminium, beschichtet wird.

Weitere bevorzugte Ausgestaltungen der vorliegenden Erfindung ergeben sich aus den abhängigen Ansprüchen und der folgenden detaillierten Beschreibung der Erfindung.

### Detaillierte Beschreibung der Erfindung

Die vorliegende Erfindung betrifft einen Glitter umfassend eine Folie auf Basis eines Polyesters, welche mit einem Metall, vorzugsweise Aluminium, beschichtet ist, bei dem die Folie im Wesentlichen antimonfrei ist. Im Wesentlichen antimonfrei bedeutet hierbei, dass die Folie einen Antimongehalt von weniger als 100 ppm, bevorzugt weniger als 50 ppm, weiter bevorzugt von weniger als 10 ppm und insbesondere bevorzugt von weniger als 5 ppm, bezogen auf das Gewicht der Folie, aufweist. Es ist jedoch nicht ausgeschlossen, dass die Folie Antimon enthält. So kann die Folie Antimon als unvermeidbare Verunreinigung in einer Menge enthalten, welche unterhalb der obigen Gehaltsgrenze liegt. Gemäß bestimmten Ausführungsformen kann die Folie zudem transparent oder im Wesentlichen transparent, beispielsweise mit einer Transmissivität gegenüber Licht im sichtbaren Bereich von 380 bis 780 nm, von 70% oder 80% oder 90% oder mehr, sein. Die Folie kann gemäß weiteren Ausführungsformen gefärbt oder ungefärbt sein, ist gemäß bestimmten Ausführungsformen jedoch ungefärbt. Zudem kann in bestimmten Ausführungsformen auf die Beschichtung mit Metall, bevorzugt Aluminium, oder die Folie und die Metallbeschichtung eine farbgebende Schicht aufgebracht werden.

Gemäß bestimmten Ausführungsformen umfasst die Folie mehr als 50 Gew.% eines Polyesters, bezogen auf das Gewicht der Folie. Geeignete Polyester umfassen hierbei Polybutylenterephthalat (PBT), Polyethylenterephthalat (PET), und Polyethylenisoterephthalat, sowie Mischungen hiervon. In bevorzugten Ausführungsformen umfasst die Folie mehr als 60 Gew.% Polyester, weiter bevorzugt mehr als 70 Gew.%, noch weiter bevorzugt mehr als 80 Gew.% und besonders bevorzugt mehr als 90 Gew.%. In bestimmten Ausführungsformen umfasst der erfindungsgemäße Glitter als Polymer nur eine Folie auf Basis eines Polyesters, welche im Wesentlichen antimonfrei ist.

Es ist hierbei anzumerken, dass sich alle Prozent-Angaben in der Beschreibung auf Gewichtsprozent beziehen, soweit dies nicht besonders angegeben ist.

Daneben kann die Folie ein oder mehr weitere Polymere, welche sich mit dem Polyester mischen oder reagieren lassen, in einer Menge von weniger als 50 Gew.%, bezogen auf das Gewicht der Folie, umfassen. Bevorzugt umfasst die Folie zusätzlich ein oder mehr (Meth)Acrylat-basiertes Polymer in einer Menge von weniger als 50 Gew.%. Der Ausdruck (Meth)Acrylat-basiertes Polymer umfasst hierbei sowohl Polymere auf Acrylatbasis wie auch Polymere auf Methacrylatbasis sowie (Co)Polymere auf Acrylat- und Methacrylatbasis wie auch Mischungen hiervon. Bevorzugt enthält die Folie weniger als 40 Gew.% des weiteren Polymers, bevorzugt auf (Meth)Acrylatbasis, weiter bevorzugt weniger als 30 Gew.%, noch weiter bevorzugt weniger als 20 Gew.% und besonders bevorzugt weniger als 10 Gew.%. Darüber hinaus kann die Folie auch Folien, die durch Co-Extrusion verschiedener Polymere hergestellt werden, sowie Folienverbunde aus verschiedenen polymeren Folien umfassen. Es ist somit nicht ausgeschlossen, dass neben der Folie auf Basis eines Polyesters weitere Polymerfolien enthalten sind, wobei bevorzugt jedoch nicht mehr als 10 Folien, weiter bevorzugt nicht mehr als 5 Folien und besonders bevorzugt nicht mehr als 3 Folien co-extrudiert oder als Folienverbund verwendet werden. Der Glitter selbst ist hierbei jedoch gemäß bestimmten Ausführungsformen im Wesentlichen antimonfrei, hat also einen Antimongehalt von weniger als 100 ppm, bevorzugt weniger als 50 ppm, weiter bevorzugt von weniger als 10 ppm und insbesondere bevorzugt von weniger als 5 ppm, bezogen auf das Gewicht des Glitters. In bevorzugten Ausführungsformen umfasst der Glitter mehr als 60 Gew.% Polyester, weiter bevorzugt mehr als 70 Gew.%, noch weiter bevorzugt mehr als 80 Gew.% und besonders bevorzugt mehr als 90 Gew.%. In bestimmten Ausführungsformen umfasst der erfindungsgemäße Glitter als Polymer nur Polyester, welcher im Wesentlichen antimonfrei ist.

Gemäß bevorzugten Ausführungsformen wird die Folie mit einem antimonfreien Katalysator hergestellt. Der antimonfreie Katalysator umfasst dabei bevorzugt mindestens ein Metall ausgewählt aus Ti, Al oder Ge. Beispiele für solche Katalysatoren sind beispielsweise aus U.K. Thiele, Chemical Fibers International, 54, S. 162-163, 2004 und U.K. Thiele, International Journal of Polymeric Materials, 50, S. 387-394, 2001 bekannt, und umfassen beispielsweise Ecocat-T der Firma Zimmer AG, TG/19 der Firma Teck Cominco, Hombifast der Firma Sachtleben Chemie, Vertec-C400 der Firma Johnson & Matthey und Tyzor der Firma DuPont.

Weiterhin ist in einem erfindungsgemäßen Glitter die Folie ferner mit einem Metall, vorzugsweise Aluminium, Silber, Gold und/oder Kupfer, bevorzugt Aluminium, beschichtet. Die Beschichtung kann hierbei gemäß bestimmten Ausführungsformen auf einer Seite, auf zwei gegenüberliegenden Seiten, auf der gesamten Folie oder in anderer Weise erfolgen. Darüber hinaus kann erfindungsgemäß in dem Glitter die Metallschicht, beispielsweise Aluminiumschicht, oder die Folie und die Metallschicht mit einer Beschichtung auf Polyurethan-, Acrylat-, Styrol-Acrylat- oder Epoxidbasis, bevorzugt Polyurethan-, Acrylat- oder Styrol-Acrylatbasis, oder einer Beschichtungslösung auf Sol-Gel-Basis beschichtet sein. Auch hier kann die Beschichtung auf einer Seite, zwei gegenüberliegenden Seiten, auf der gesamten Metallschicht, beispielsweise Aluminiumschicht, der Folie und der Metallschicht, beispielsweise Aluminiumschicht, oder auf andere Weise vorhanden sein. Insbesondere ist bei solchen beschichteten Folien eine gleichmäßigere und glattere Oberfläche des Glitters beobachtbar, die sich beispielsweise auch in einer weiter verbesserten Brillanz niederschlägt. Auch wird für Glitter umfassend solche beschichteten Folien ein besseres Skin-Feeling erhalten. Es hat sich zudem gezeigt, dass bei der Herstellung solcher beschichteter Glitter, insbesondere auch bei Verwendung eines Wirbelschichtverfahrens, weniger Glitter an beispielsweise der Behälterwand des Behälters zur Herstellung anhaften, was zu einer verbesserten, einheitlicheren Beschichtungsqualität der Glitter führt. An der Behälterwand haftende Glitter werden hingegen nicht oder nicht ausreichend beschichtet. Es ergibt sich somit bei der Herstellung solcher beschichteter Glitter auch ein effizienteres und besseres Herstellungsverfahren.

Darüber hinaus kann der erfindungsgemäße Glitter auch weitere farb- und/oder effektgebende Schichten umfassen, wie sie dem Fachmann bekannt sind und die ein Fachmann geeignet beschichten kann, beispielsweise aus der Gasphase oder aus Flüssigkeit/Lösung. Gemäß bevorzugten Ausführungsformen ist der erfindungsgemäße Glitter kein irisierender Glitter. Insbesondere ist für solche irisierende Glitter keine Metallbeschichtung vorgesehen, die den irisierenden Effekt des Glitters beeinträchtigen kann, so dass die erfindungsgemäße Metallbeschichtung nachteilig für irisierende Glitter sein kann. Zudem umfassen irisierende Glitter im Allgemeinen mehrere 100 Einzellagen an Folien, welche den Iriseffekt bedingen, was eine komplizierte Herstellung bedingt. Auch sind irisierende Glitter zu teuer. Zudem kann durch die Vielzahl an Schichten und der fehlenden Metallisierung nicht gewährleistet werden, dass der erfindungsgemäße Effekt einer verbesserten Brillanz und eines verbesserten Skin-Feelings, sowie eines verbesserten Beschichtungsverhaltens erzielt werden kann. Insbesondere ergibt sich durch die fehlende Metallisierung eine geringere Brillianz, und durch dickere Folienstärken ergibt sich insbesondere ein schlechteres Skinfeeling.

Gemäß bevorzugten Ausführungsformen weist die Folie, die bei der Herstellung des Glitters verwendet wird und die somit im Glitter enthalten ist, eine Dicke von mehr als 5 µm, bevorzugt von mehr als 10 µm, weiter bevorzugt von mehr als 12 µm auf. Insbesondere bei diesen im Vergleich zum Stand der Technik dicken Folien können im erfindungsgemäßen Glitter eine weiter verbesserte gleichmäßige und glatte Oberfläche des Glitters und eine verbesserte Brillanz erhalten werden. Weiterhin ist die Dicke bevorzugt weniger als 50 µm, beispielsweise weniger als 40 µm, bevorzugt weniger als 30 µm, weiter bevorzugt weniger als 28 µm und besonders bevorzugt weniger als 25 µm, beispielsweise auch weniger als 20 µm.

Darüber hinaus offenbart ist ein Verfahren zur Herstellung eines Glitters, bei dem eine Folie auf Basis eines Polyesters mit einem antimonfreien Katalysator hergestellt wird, die Folie mit einem Metall, vorzugsweise Aluminium, beschichtet wird, und die Folie danach geschnitten wird, oder die Folie geschnitten wird und danach mit einem Metall, vorzugsweise Aluminium, beschichtet wird. Bevorzugt ist, dass die Folie zunächst mit einem Metall, vorzugsweise Aluminium, beschichtet wird, und die Folie danach geschnitten wird. Nach dem Schneiden der Folie ergeben sich somit Partikel. Die Schnittform der Folie ist nicht beschränkt, und die Partikel können beispielsweise in einer hexagonähnlichen Form geschnitten werden, also einer Form, die einem Hexagon gleicht. Bei einer Verwendung solch geschnittener Partikel ergeben sich folglich hexagonähnliche Glitter, jedoch können auch quadratische, runde oder rechteckige Glitter oder Glitter irgendeiner anderen Form durch Schneiden einer entsprechenden Form hergestellt werden. Die Partikelgröße ist hierbei nicht beschränkt und kann beispielsweise eine Diagonallänge (beispielsweise für hexagonähnliche Glitter) bzw. Länge oder einen Durchmesser von 50 µm und mehr, 100 µm und mehr, oder 150 µm und mehr umfassen.

Beispielsweise können die erfindungsgemäßen Glitter zudem eine Größe (Diagonallänge, Länge bzw. Durchmesser oder ähnliches) von 500 µm oder weniger aufweisen. Die Folie kann hierbei eine Dicke von mehr als 5 µm, bevorzugt von mehr als 10 µm, weiter bevorzugt von mehr als 12 µm aufweisen und die oben genannte Zusammensetzung aufweisen. Weiterhin ist die Dicke bevorzugt weniger als 50 µm, beispielsweise weniger als 40 µm, bevorzugt weniger als 30 µm, weiter bevorzugt weniger als 28 µm und besonders bevorzugt weniger als 25 µm, beispielsweise auch weniger als 20 µm. Als antimonfreie Katalysatoren kommen die oben beschriebenen in Betracht.

Die Folie wird erfindungsgemäß mit einem Metall, vorzugsweise Aluminium, beschichtet. Die Beschichtung kann auf einer Seite, auf zwei gegenüberliegenden Seiten, auf der gesamten Folie oder in anderer Weise erfolgen. Verfahren zur Beschichtung der Folien sind dem Fachmann bekannt. Beispielsweise kann eine Beschichtung durch Bedampfen, durch Aufsprühen eines Aerosols, Lackieren, oder auf andere Weise, bevorzugt durch Bedampfen, erfolgen. Die Beschichtung mit Metall kann vor oder nach dem Schneiden der Folie erfolgen. Bevorzugt erfolgt die Metallisierung vor dem Schneiden. Wenn die Metallisierung vor dem Schneiden erfolgt, kann in einem zweiten Beschichtungsschritt, beispielsweise durch Lackieren, Tiefdruck, etc., eine Polymerbeschichtung, beispielsweise auf Polyurethan-, Acrylat-, Styrol-Acrylat- oder Epoxidbasis, aufgebracht werden. Eine solche Polymerbeschichtung der metallisierten Folie erleichtert den Schneidvorgang und führt zu einer verbesserten Herstellung von Partikeln durch den Schneidvorgang.

Weiterhin können in einem erfindungsgemäßen Verfahren die Metallschicht, beispielsweise eine Aluminiumschicht, oder die Folie und die Metallschicht, welche gegebenenfalls mit einer Polymerschicht beschichtet ist/sind, nach dem Schneiden mit einer Beschichtung auf Polyurethan-, Acrylat-, Styrol-Acrylat- oder Epoxidbasis beschichtet werden. Die Beschichtung mit einem Polymer auf Polyurethan-, Acrylat-, Styrol-Acrylat- oder Epoxidbasis nach dem Schneiden der Folie kann beispielsweise durch Einbringen in eine Polymerlösung oder durch Aufdampfen erfolgen. Bevorzugt erfolgt die Beschichtung nach dem Schneiden mit einem Polymer auf Polyurethan-, Acrylat-, Styrol-Acrylat- oder Epoxidbasis oder einer Beschichtungslösung auf Sol-Gel-Basis durch ein Wirbelschichtverfahren, wie es beispielsweise in DE 102010001971 A1 offenbart ist. Geeignete Beschichtungstemperaturen liegen hier beispielsweise bei 40 bis 140°C, bevorzugt 50 bis 100°C, weiter bevorzugt 60 bis 80°C.

Bei dem Wirbelschichtverfahren werden grundsätzlich zum Zwecke der Beschichtung von Partikeln die einzelnen Partikel mit einer Suspension, Dispersion oder Lösung beschichtet. Beim Wirbelschichtverfahren, welches an sich bekannt ist, werden Granulate oder Partikel durch einen Luftstrom aufgewirbelt, wobei der Luftstrom einen Primärstrom darstellt und das für die Partikel vorgesehene Beschichtungsmaterial über einen Sekundärstrom eingeführt wird, sodass das Beschichtungsmaterial, welches in Form einer Suspension oder Dispersion vorliegt, die einzelnen Partikel vollständig über die gesamte Außenfläche überzieht. Wirbelschichtverfahren und Vorrichtungen zur Durchführung von Wirbelschichtverfahren sind z.B. bekannt aus der Veröffentlichung Betriebsanleitung "Mini Glatt mit Microkit", Okt. 2003, sowie aus der US 3,241,520 und US 5,632,102.

Die Beschichtung mittels der Wirbelschicht kann gegebenenfalls wiederholt werden, um eine mehrfache Beschichtung der Glitter vorzunehmen, um beispielsweise veränderte chemische oder physikalische Eigenschaften der Beschichtungsschicht zu erhalten bzw. unterschiedliche Schutzeigenschaften zu ermöglichen.

Weiterhin offenbart ist die Verwendung der erfindungsgemäßen Glitter in kosmetischen Produkten. Kosmetische Produkte umfassen hierbei beispielsweise Pasten, Salben, Cremes, Nagellack, Puder, flüssige Eyeliner, usw., welche die Glitter in üblichen Mengen in den Formulierungen enthalten können. Darüber hinaus betrifft die vorliegende Erfindung auch kosmetische Produkte, welche die erfindungsgemäßen Glitter enthalten.

Nachfolgend wird die Erfindung näher erläutert anhand bevorzugter Ausführungsformen.

Die Figuren zeigen Schnittansichten durch Glitter mit dem erfindungsgemäßen Aufbau. Hierbei zeigen:

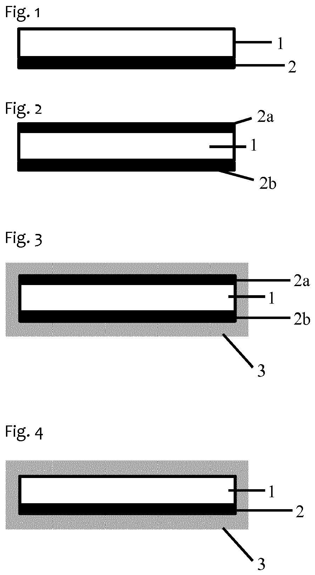

- Figuren 1 bis 4:: Schematische Schnittansichten eines erfindungsgemäßen Glitters mit unterschiedlichem Schichtaufbau

Erfindungsgemäße Glitter umfassen beispielsweise Glitterpartikel mit der Größe von 20 µm bis 500 µm und einer Dicke von 25 µm, die gemäß einer ersten bevorzugten Ausführungsform, die in Fig. 1 dargestellt ist, aus einer transparenten, im Wesentlichen antimonfreien Polyesterfolie 1 bestehen, welche einseitig mit einer Aluminiumschicht 2 beschichtet ist, und durch Schneiden der beschichteten antimonfreien Polyesterfolie gewonnen werden. Eine Beschichtung aus Aluminium wird hierbei vorzugsweise unter Vakuum aufgedampft. Gemäß einer weiteren Ausführungsform, die in Figur 2 dargestellt ist können die im Wesentlichen antimonfreien Polyesterfolienpartikel 1 beidseitig mit Aluminiumschichten 2a, 2b beschichtet sein. Eine Beschichtung aus Aluminium wird hierbei vorzugsweise unter Vakuum aufgedampft. Darüber hinaus können die beidseitig mit Aluminiumschichten 2a, 2b beschichteten, im Wesentlichen antimonfreien Polyesterfolienpartikel 1 in einer weiteren Ausführungsform, wie in Fig. 3 gezeigt, zusätzlich auf allen Seiten gleichmäßig mit einer Schicht aus Polyurethan 3 beschichtet sein. Alternativ können im Wesentlichen antimonfreie Polyesterfolienpartikel, welche einseitig mit einer Aluminiumschicht 2 beschichtet sind, in einer weiteren Ausführungsform, wie in Fig. 4 gezeigt, auf allen Seiten gleichmäßig mit einer Schicht aus Polyurethan 3 beschichtet sein.

In den Figuren 1 bis 4 kann die im Wesentlichen antimonfreien Polyesterfolie gemäß alternativen Ausgestaltungen auch gefärbt sein oder die Glitterpartikel können nach dem Aufbringen der Metallschicht gefärbt werden.

Die Beschichtung mit Polyurethan 3 in Figuren 3 und 4 kann beispielsweise gemäß einem Wirbelschichtverfahren hergestellt werden.

Generell werden hierbei die unterschiedlichen Ausgangspartikel unter vergleichbaren Parametern behandelt und in dem Wirbelschichtverfahren mit dem Schutzschichtmaterial vollständig umgeben, sodass alle Schnittkanten bzw. Bruchkanten der Ausgangspartikel eingeschlossen werden. Die genaue Anpassung der Parameter während des Wirbelschichtverfahrens ergibt sich aus den Dichten und Größen der jeweiligen Materialien. In den Figuren 3 und 4 sind sämtlichen Außenflächen der Partikel, mit oder ohne Aluminiumschicht, mit einer Polyurethanschicht umgeben.

Beispielsweise können unter Verwendung einer Wirbelschichtanlage WFP-8-Anlage der Firma DMR Prozesstechnologie GmbH (Schweiz) fünf Kilogramm Glitterpartikel aus im Wesentlichen antimonfreiem Polyester, welche mit Aluminium beschichtet sind, mit einer Polyurethanschicht beschichtet werden. Die Partikel aus im Wesentlichen antimonfreien Polyester wurden hierbei aus einer im Wesentlichen antimonfreien Polyesterfolie mit einer Dicke von 25 µm hergestellt, die mit Aluminium bedampft und anschließend rot gefärbt wurde. Die Partikel wurden aus dieser Folie als z.B. hexagonale Teilchen mit der Größe 200 µm, gemessen über die Länge der zueinander parallelen Schnittkanten, geschnitten. Die Stärke der Teilchen beträgt im Durchschnitt 25-35µm. Diese Partikel wurden in der genannten Anlage mittels einer auf 60°C vorgewärmter Prozessluft mit einem Luftdurchsatz von 60 m³/h verwirbelt zur Erzeugung eines sogenannten Mischbettes, dem mittels Bottom-Spray, d.h. von unten her, eine wässrige Polyurethanlösung zugeführt wird.

Der Primärdruck (Prozessluft) und der Sekundärdruck wurden im Verhältnis 1 : 2,5 eingestellt, um eine gute Benetzung des gesamten Mischbettes und damit der im Mischbett vorhandenen Glitterpartikel zu erzielen. Hierbei wurden die Tröpfchen der Polyurethanlösung fein verteilt und gleichmäßig in das Wirbelbett eingeblasen. Bei diesem Beispiel wurden 1000 ml Polyurethanlösung innerhalb von 60 Minuten eingesprüht, um einer schnellen Antrocknung der Tröpfchen auf den Partikeln entgegenzuwirken. Nach Ablauf dieser Zeit konnten 5 kg beschichteter Glitter der Anlage entnommen werden, und durch eine anschließende 30-minütige Temperung bei 120°C wurde die Beschichtung stabil vernetzt. Hierbei ergab sich eine homogene Beschichtung sämtlicher Glitterpartikel.

Abhängig von der Partikelgröße und Partikeldichte der zu erzeugenden Glitter sind unterschiedliche Fluidisierungsgrade innerhalb der Wirbelschicht einzustellen. Abhängig vom Gewicht der Glitter wird bei größeren Glitterpartikeln ein höherer Prozessdruck sowohl hinsichtlich der die Verwirbelung erzeugenden Prozessluft als auch hinsichtlich der in das Wirbelbett eingeblasenen Polymerlösung erforderlich als bei kleineren die Glitter bildenden Partikeln. Es ist eine große Ausdehnung der Wirbelschicht erforderlich, um einen hinreichend großen Abstand zwischen den einzelnen Glitterpartikeln zu gewährleisten, damit die einzelnen Glitterpartikel, getrennt zugänglich, mit dem Schutzschichtmaterial beschichtet werden können und andererseits die Partikel während der Beschichtung nicht zusammenkleben.

Als Beschichtungsmaterial lässt sich anstelle einer Polyurethanlösung, auch eine wässrige Acrylat- oder Styrol-Acrylat -Lösung, eine Beschichtungslösung auf Sol-Gel-Basis oder eine wässrige Epoxidlösung einsetzen.

Die Erzeugung der Wirbelschicht erfolgt durch Düsen, die einen Primärstrom erzeugen, wobei der Druck dieses Primärstromes die Länge des Sprühkegels bestimmt, und durch einen Sekundärstrom, dessen Druck die Breite des Sprühkegels festlegt.

Es hat sich herausgestellt, dass je kleiner die Lösungstropfen bzw. je feiner der Sprühnebel sein soll, umso größer der Primärdruck einzustellen ist. Bei kleineren Partikeln sollten die Tropfen möglichst fein sein. Die Trocknung kleiner Tropfen erfolgt jedoch schneller als bei größeren Tropfen, sodass bei der Erzeugung kleinerer Tropfen mit einer größeren Menge an z. B. wässriger Polyurethanlösung gearbeitet werden muss, damit die Feuchtigkeit in der Wirbelschicht gleich ist.

Alternativ zu im Boden angeordneten Düsen (Bottom-Spray) können diese Düsen auch an anderer Stelle innerhalb der Anlage vorgesehen werden, z. B. im oberen Bereich der Anlage oder im seitlichen Bereich (Top-Spray, Tangential-Spray).

Die Zugabe des Beschichtungsmaterials in Form von Polyurethan-Lösungen oder anderen erfindungsgemäßen Lösungen erfolgt durch ein zeitlich kontrolliertes Dosiersystem, beispielsweise eine Schlauchpumpe. Die Trocknung oder Vernetzung erfolgt abhängig vom Lösungsmittel über die Erwärmung der Prozessluft oder Beheizen des Misch- bzw. Wirbelbettes. Im Bedarfsfall können die Glitter anschließend einer zusätzlichen thermischen Bearbeitung unterworfen werden, beispielsweise in Form einer Polymerisation der Schutzschicht bei Temperaturen höher als 50°C.

Gegebenenfalls kann eine Anpassung hinsichtlich der Temperaturen notwendig sein, wie auch hinsichtlich der Parameter, mit welchen das Wirbelschichtverfahren durchgeführt wird.

Die Beschichtung mittels der Wirbelschicht kann gegebenenfalls wiederholt werden, um eine mehrfache Beschichtung der Glitterpartikel vorzunehmen, um beispielsweise veränderte chemische oder physikalische Eigenschaften der Schutzschicht zu erhalten bzw. unterschiedliche Schutzeigenschaften zu ermöglichen.

Durch das vorstehend beschriebene Wirbelschichtverfahren wird der mehrschichtige Aufbau mit einer Beschichtungsschicht versehen, die sämtliche Oberflächen des Glitterpartikels überzieht.

Abhängig von den Erfordernissen können Farbanteile, beispielsweise in Form von Farbpigmenten, entweder in die Beschichtungsschicht oder auch vorher in Verbindung mit dem Aufbringen anderer Schichten eingebracht werden, um an der betreffenden Fläche eine Einfärbung vorzusehen.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss anhand einiger beispielhafter Ausführungsformen dargestellt, die diese jedoch nicht einschränken.

Partikel aus im Wesentlichen antimonfreien Polyester wurden aus einer im Wesentlichen antimonfreien Polyesterfolie mit einer Dicke von 23 µm hergestellt, die mit Aluminium bedampft und mit einer acrylatbasierten Polymerbeschichtung beschichtet wurde. Die Partikel wurden aus dieser Folie als hexagonale Teilchen mit Größen von 150 µm (Beispiel 1), 200 µm (Beispiel 2), und 400 µm (Beispiel 3), gemessen über die Länge der zueinander parallelen Schnittkanten, geschnitten. Die Stärke der Teilchen beträgt im Durchschnitt 25-35µm.

Die gemäß Beispiel 3 aus antimonfreier Folie hergestellter Glitter und ein herkömmlicher, antimonhaltiger Glitter (PolyFlake, silver ctd., 0.015, Glitterex Corp., USA, Vergleichsbeispiel 3) wurden hinsichtlich ihrer Formbeständigkeit geprüft. Beide Glitter weisen eine Foliendicke von 23 µm auf eine Partikelgröße von 400 µm.

Daneben wurden Glitter der Partikelgrößen 150 µm und 200 µm hinsichtlich der Formbeständigkeit/Formstabilität bei Temperaturbelastung untersucht. Bei allen Prüfungen wurde jeweils eine Doppelbestimmung durchgeführt.

Die Formstabilität bei Temperaturbelastung wird geprüft, indem die Glitterprobe in einem Wärmeschrank für eine festgelegte Zeit einer bestimmten Temperatur ausgesetzt wird.

Es wurden folgende Prüfparamter verwendet.
1. Temperaturbelastung 15 Minuten bei 175 °C
2. Temperaturbelastung 5 Minuten bei 230 °C

Die Bestimmung der Formbeständigkeit erfolgte hierbei mittels Lichtmikroskop bei einer 25-fachen bis zu 200-fachen Vergrößerung.

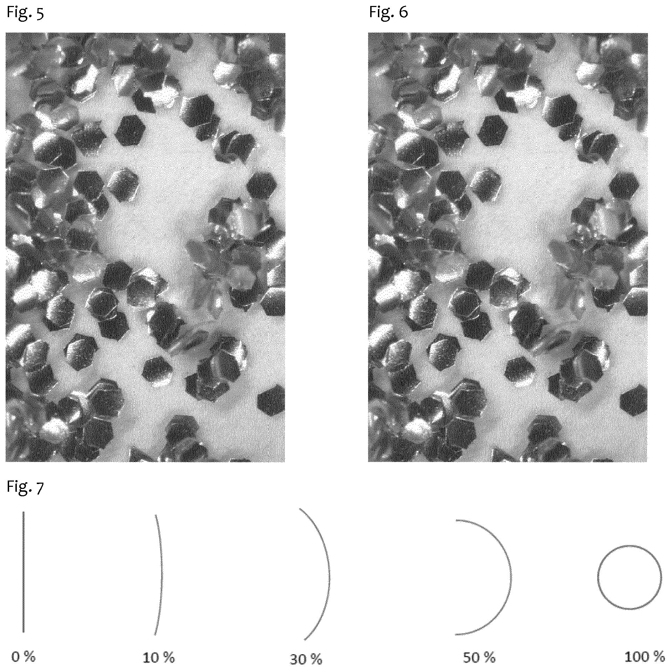

Ein visueller Vergleich von Beispiel 3 und dem Vergleichsbeispiel unter einer Temperaturbelastung von 230°C für 5 Minuten ist den lichtmikroskopischen Aufnahmen in Figur 5 (Beispiel 3) und Figur 6 (Vergleichsbeispiel 3) zu entnehmen. Bereits in diesen Bildern ist die deutliche Wölbung/Verformung der Partikel im Vergleichsbeispiel ersichtlich. Es hat sich gezeigt, dass sich antimonfreie Glitter durch eine bessere Formbeständigkeit bei Temperaturbelastung auszeichnen. Formbeständigkeit bedeutet in diesem Zusammenhang, dass die Glitterpartikel ihre plättchenartige, flache Form behalten und sich nicht bei Temperaturbelastung biegen und keine gewölbte Form annehmen.

Bei einer weiteren Prüfung zur Quantifizierung der Formbeständigkeit wurden Glitterpartikel jeweils den in Tabelle 1 angegebenen Temperaturen und Zeitdauern ausgesetzt.

Um die Formbeständigkeit zu quantifizieren, wurde diese hierbei anhand der lichtmikroskopischen Aufnahmen gemäß folgender Bewertungsskala empirisch bewertet: 0 : keine Veränderung (Verformung 0 - 10%)
1: geringe Verformung / akzeptabel (Verformung 10 - 30%)
2: deutliche Verformung / nicht akzeptabel (Verformung 30 - 50%)
3: Formverlust / nicht akzeptabel (Verformung > 50%)

Die Kriterien für die Verformung sind hierbei Figur 7 zu entnehmen.

**Tabelle 1: Prüfergebnisse bezüglich Temperaturbeständigkeit**

| Prüfparameter | Verformung von Glitter gemäß Beispiel 3, antimonfrei hergestellt | Verformung von Glitter gemäß dem Vergleichsbeispiel, antimonhaltig |
|---|---|---|
| 175°C / 15 Minuten | 0 | 1 bis 2 |
| 230°C / 30 Minuten | 1 | 2 |

Der deutlichste Effekt zeigte sich bei der Größe von 400 µm (Beispiel 3), wobei auch in den anderen Beispielen entsprechende Ergebnisse erhalten wurden.

Darüber hinaus war die stärkere Verformung nicht nur visuell erkennbar, sondern zeigte sich auch in der haptischen Wahrnehmung. Der eher dreidimensionale Charakter von gewölbten Partikeln ist in vielen Anwendungen nicht erwünscht, weil dadurch die Glitter rauer wirken und eine ungleichmäßige Oberflächenstruktur erzeugen. Die Formstabilität ist somit auch bezüglich der haptischen Wahrnehmung ein wichtiges Qualitätskriterium.

Darüber hinaus ist die plättchenartige, flache Partikelform Grundlage dafür, dass Glitter einen spiegelartigen Glanzeffekt zeigen und somit als Effektmaterial Verwendung finden. Durch eine gewölbte Partikelform ist die Spiegelwirkung vermindert, wodurch der gewünschte Glitzer-Effekt weniger ausgeprägt erscheint.

Auch dies konnte messtechnisch in Bezug auf die Brillanz / Helligkeit des antimonfrei hergestellten Glitters belegt werden anhand von Farbmessungen im L*a*b*-Farbraum, bei denen der Messwert dL* ein Maß für die Helligkeit darstellt. Hierfür wurde ein Spectrophotometer CM-3500d, Konica-Minolta, Japan (Messparameter: Messgeometrie d/8°, Lichtart D65, Normalbetrachter 10°) verwendet.

In den Messungen wurden die Glitter gemäß Beispielen 1 und 2 mit antimonhaltigen Glittern mit Größen von 150 µm (PolyFlake, silver ctd., 0.006, Glitterex Corp., USA, Vergleichsbeispiel 1) und 200 µm (PolyFlake, silver ctd., 0.008, Glitterex Corp., USA, Vergleichsbeispiel 2) verglichen.

Bei den Messungen ergaben sich positive dL*-Werte bei dem Vergleich gemäß nachfolgender Tabellen 2 und 3, welche die Messergebnisse bei Glitterproben der Partikelgröße 200 µm und der Partikelgröße 150 µm zeigen. Das bedeutet, dass der antimonfrei hergestellte Glitter farblich heller erscheint als ein vergleichbarer, antimonhaltiger Glitter bei gleicher Partikelgröße.

**Tabelle 2: Prüfergebnisse bezüglich Helligkeit**

| | Glitter von Vergleichsbeispiel 2 | Glitter von Beispiel 2 | Unterschied von Beispiel 2 zu Vergleichsbeispiel 2 |
|---|---|---|---|
| L* | 58,74 | 64,53 | dL* 5,80 |
| a* | -0,64 | -0,71 | da* -0,05 |
| b* | -1,73 | -0,90 | db* 0,83 |

**Tabelle 3: Prüfergebnisse bezüglich Helligkeit**

| | Glitter von Vergleichsbeispiel 1 | Glitter von Beispiel 1 | Unterschied von Beispiel 1 zu Vergleichsbeispiel 1 |
|---|---|---|---|
| L* | 61,25 | 64,22 | dL* 2,97 |
| a* | -0,66 | -0,92 | da* -0,26 |
| b* | -1,55 | -1,17 | db* 0,38 |

Ein weiterer Vorteil ergibt sich hinsichtlich der Temperaturbeständigkeit der antimonfrei hergestellten Glitter.

Es wurde die Farbveränderung von Glitterproben bei Temperaturbelastung (Wärmeschrank (120°C / 120 Minuten)) untersucht. Nachfolgende Tabelle 4 zeigt die Ergebnisse des antimonfrei hergestellten Glitters des Beispiels 1 im Vergleich zu zwei Proben von handelsüblichen, antimonhaltigen Glittern mit einer Partikelgröße von 150 µm (Vergleichsbeispiele 1 und 1' (1SHB Silver, 0.006, Advance Syntex (P) Ltd., India).

Der Wert dE*ab ist hierbei ein Maß für den Farbunterschied (Farbabstand) zweier Proben, vorliegend der Probe jeweils vor und nach der Temperaturbelastung. Je kleiner dieser Wert ist, desto farbähnlicher sind zwei Proben. Der antimonfrei hergestellte Glitter des Beispiels 1 zeigt die geringste Farbveränderung bei Temperaturbelastung, der dE*ab-Wert ist am geringsten.

**Tabelle 4: Farbveränderung bei Temperaturbelastung**

| | Glitter gemäß Beispiel 1 | Glitter gemäß Vergleichsbeispiel 1 | Glitter gemäß Vergleichsbeispiel 1' |
|---|---|---|---|
| Farbveränderung dE*ab | 0,45 | 1,70 | 1,43 |

Mit den erfindungsgemäßen antimonfreien Glittern können eine bessere Formstabilität bei Temperaturbelastung, Brillanz und Temperaturbeständigkeit erreicht werden, was so nicht zu erwarten war. Somit erschließen sich für den antimonfreien Glitter auch Anwendungsfelder, die eine verbesserte Formstabilität bei Temperaturbelastung erfordern.

## Patentansprüche

1. Glitter umfassend eine Folie auf Basis eines Polyesters, welche mit einem Metall, vorzugsweise Aluminium, beschichtet ist, wobei die Folie einen Antimongehalt von weniger als 100 ppm, bezogen auf das Gewicht der Folie, aufweist.

2. Glitter gemäß Anspruch 1, wobei die Folie mit einem antimonfreien Katalysator hergestellt wird.

3. Glitter gemäß einem der vorigen Ansprüche, wobei die Metallschicht, beispielsweise eine Aluminiumschicht, oder die Folie und die Metallschicht mit einer Beschichtung auf Polyurethan-, Acrylat-, Styrol-Acrylat- oder Epoxidbasis oder einer Beschichtung basierend auf einer Beschichtungslösung auf Sol-Gel-Basis beschichtet ist.

4. Glitter gemäß einem der vorigen Ansprüche, wobei die Folie mehr als 50 Gew.% eines Polyesters umfasst.

5. Glitter gemäß einem der vorigen Ansprüche, wobei die Folie zusätzlich ein (Meth)Acrylat-basiertes Polymer in einer Menge von weniger als 50 Gew.% umfasst.

6. Glitter gemäß einem der vorigen Ansprüche, wobei die Folie eine Dicke von mehr als 5 µm aufweist.

7. Glitter gemäß einem der vorigen Ansprüche, wobei der antimonfreie Katalysator mindestens ein Metall ausgewählt aus Ti, Al oder Ge umfasst.

8. Verfahren zur Herstellung eines Glitters, bei dem eine Folie auf Basis eines Polyesters mit einem antimonfreien Katalysator hergestellt wird, mit einem Metall, vorzugsweise Aluminium, beschichtet wird und die Folie danach geschnitten wird, oder die Folie geschnitten wird und danach mit einem Metall, vorzugsweise Aluminium, beschichtet wird.

9. Verfahren gemäß Anspruch 8, wobei die Folie eine Dicke von mehr als 5 µm aufweist.

10. Verfahren gemäß Anspruch 8 oder 9, wobei die Folie mehr als 50 Gew.% eines Polyesters umfasst.

11. Verfahren gemäß einem der Ansprüche 8 bis 10, wobei die Folie zusätzlich ein (Meth)Acrylat-basiertes Polymer in einer Menge von weniger als 50 Gew.% umfasst.

12. Verfahren gemäß einem der Ansprüche 8 bis 11, wobei der antimonfreie Katalysator mindestens ein Metall ausgewählt aus Ti, Al oder Ge umfasst.

13. Verfahren gemäß einem der Ansprüche 8 bis 12, wobei die Folie oder die Metallschicht, beispielsweise eine Aluminiumschicht, oder die Folie und die Metallschicht mit einer Beschichtung auf Polyurethan-, Acrylat-, Styrol-Acrylat- oder Epoxidbasis oder einer Beschichtung basierend auf einer Beschichtungslösung auf Sol-Gel-Basis beschichtet wird.

14. Verwendung von Glitter gemäß einem der Ansprüche 1 - 7 in kosmetischen Produkten.

15. Kosmetisches Produkt, umfassend einen Glitter gemäß einem der Ansprüche 1 - 7.

## Claims

1. Glitter comprising a film based on a polyester, which is coated with a metal, preferably aluminium, wherein the film has an antimony content of less than 100 ppm based on the weight of the film.

2. Glitter according to claim 1, wherein the film is manufactured using an antimony-free catalyst.

3. Glitter according to any of the preceding claims, wherein the metal layer, for example an aluminium layer, or the film and the metal layer are coated with a coating based on polyurethane, acrylate, styrene acrylate or epoxide or a coating based on a coating solution based on sol gel.

4. Glitter according to any of the preceding claims, wherein the film comprises more than 50 % by weight of a polyester.

5. Glitter according to any of the preceding claims, wherein the film additionally comprises a (meth)acrylate-based polymer in an amount of less than 50 % by weight.

6. Glitter according to any of the preceding claims, wherein the film has a thickness of more than 5 µm.

7. Glitter according to any of the preceding claims, wherein the antimony-free catalyst comprises at least one metal selected from Ti, Al or Ge.

8. Method for manufacturing a glitter, in which a film based on a polyester is manufactured using an antimony-free catalyst and is coated with a metal, preferably aluminium, and the film is subsequently cut, or the film is cut and subsequently coated with a metal, preferably aluminium.

9. Method according to claim 8, wherein the film has a thickness of more than 5 µm.

10. Method according to either claim 8 or claim 9, wherein the film comprises more than 50 % by weight of a polyester.

11. Method according to any of claims 8 to 10, wherein the film additionally comprises a (meth)acrylate-based polymer in an amount of less than 50 % by weight.

12. Method according to any of claims 8 to 11, wherein the antimony-free catalyst comprises at least one metal selected from Ti, Al or Ge.

13. Method according to any of claims 8 to 12, wherein the film or the metal layer, for example an aluminium layer, or the film and the metal layer are coated with a coating based on polyurethane, acrylate, styrene acrylate or epoxide or a coating based on a coating solution based on sol gel.

14. Use of glitter according to any of claims 1 - 7 in cosmetic products.

15. Cosmetic product comprising a glitter according to any of claims 1 - 7.

## Revendications

1. Paillette comprenant une feuille à base d'un polyester, laquelle est revêtue d'un métal, de préférence, d'aluminium, dans laquelle la feuille présente une teneur en antimoine inférieure à 100 ppm, par rapport au poids de la feuille.

2. Paillette selon la revendication 3, dans laquelle la feuille est fabriquée avec un catalyseur exempt d'antimoine.

3. Paillette selon l'une des revendications précédentes, dans laquelle la couche métallique, par exemple, une couche d'aluminium, ou la feuille et la couche métallique, est revêtue avec un revêtement à base de polyuréthane, d'acrylate, de styrène-acrylate ou d'époxyde, ou un revêtement basé sur une solution de revêtement à base sol-gel.

4. Paillette selon l'une des revendications précédentes, dans laquelle la feuille comprend plus de 50 % en poids d'un polyester.

5. Paillette selon l'une des revendications précédentes, dans laquelle la feuille comprend en outre un polymère à base de (méth)acrylate en une quantité inférieure à 50 % en poids.

6. Paillette selon l'une des revendications précédentes, dans laquelle la feuille présente une épaisseur supérieure à 5 µm.

7. Paillette selon l'une des revendications précédentes, dans laquelle le catalyseur exempt d'antimoine comprend au moins un métal choisi parmi le Ti, l'Al ou le Ge.

8. Procédé de fabrication d'une paillette, dans lequel une feuille à base d'un polyester est fabriquée avec un catalyseur exempt d'antimoine, revêtue avec un métal, de préférence, l'aluminium, et la feuille est ensuite découpée, ou la feuille est découpée et ensuite revêtue avec un métal, de préférence, l'aluminium.

9. Procédé selon la revendication 8, dans lequel la feuille présente une épaisseur supérieure à 5 µm.

10. Procédé selon la revendication 8 ou 9, dans lequel la feuille comprend plus de 50 % en poids d'un polyester.

11. Procédé selon l'une des revendications 8 à 10, dans lequel la feuille comprend en outre un polymère basé sur un (méth)acrylate dans une quantité inférieure à 50 % en poids.

12. Procédé selon l'une des revendications 8 à 11, dans lequel le catalyseur exempt d'antimoine comprend au moins un métal, choisi parmi le Ti, l'Al ou le Ge.

13. Procédé selon l'une des revendications 8 à 12, dans lequel la feuille, ou la couche métallique, par exemple, une couche d'aluminium, ou la feuille et la couche métallique, est (sont) revêtue (revêtues) avec un revêtement à base de polyuréthane, d'acrylate, de styrène-acrylate ou d'époxyde, ou un revêtement se basant sur une solution de revêtement à base sol-gel.

14. Utilisation de paillettes selon l'une des revendications 1 à 7 dans des produits cosmétiques.

15. Produit cosmétique comprenant des paillettes selon l'une des revendications 1 à 7.
